# EUROPEAN PATENT SPECIFICATION

(11) **EP 4 429 528 B1**
(45) Date of publication and mention of the grant of the patent: **12.11.2025**
(21) Application number: 22838800.5
(22) Date of filing: 16.12.2022
(51) Int. Cl.: A47J 31/44, A23L 2/38, A23P 30/40, A23B 11/13

(54) **METHOD FOR TREATING MILK OR A PLANT-BASED BEVERAGE AND DEVICE IMPLEMENTING SAID TREATMENT METHOD**
VERFAHREN ZUR BEHANDLUNG VON MILCH ODER EINEM GETRÄNK AUF PFLANZENBASIS UND VORRICHTUNG ZUR DURCHFÜHRUNG DES BESAGTEN BEHANDLUNGSVERFAHRENS
PROCÉDÉ DE TRAITEMENT DU LAIT OU D'UNE BOISSON À BASE DE PLANTE ET DISPOSITIF METTANT EN OEUVRE LEDIT PROCÉDÉ DE TRAITEMENT

(30) Priority: 23.12.2021 IT 202100032594
(43) Date of publication of application: 18.09.2024
(73) Proprietor: De' Longhi Appliances S.r.l., 31100 Treviso (IT)
(72) Inventor: PANCIERA, Antonio, 31100 Treviso (IT); BENEDETTI, Alessandro, 31100 Treviso (IT); ROSETTA, Simone, 31100 Treviso (IT)
(74) Representative: Rapisardi, Mariacristina
(86) International application number: PCT/EP2022/086283
(87) International publication number: WO 2023/117736

(56) References cited:
- EP-A1- 3 332 678
- EP-A1- 3 763 258
- EP-A1- 3 821 770
- US-A1- 2020 270 114

## Description

The present invention relates to a method for treating milk or a plant-based beverage, and to a device, particularly but not necessarily a coffee machine, which implements said treatment method.

It is well known that many models of automatic coffee machines are also capable of frothing and heating milk, for example to make a cappuccino.

The result in the cup largely depends on the physicochemical properties of the milk used.

As is well known, today various types of milk of animal or plant origin can be used to produce a beverage according to consumer tastes.

In general, the known coffee machines are set with standard parameters for frothing and heating that do not take into account, therefore, the type of milk used, with the inevitable consequence that the result in the cup is extremely variable and does not always meet demands.

EP3821770 A1 discloses the relevant background art of the present invention.

EP3332678 A1 describes a spectrometric sensor used only to discriminate whether the milk has deteriorated and is thus no longer fit to be consumed.

The technical task of the present invention, therefore, is to provide a method for treating milk or a plant-based beverage that enables the aforementioned technical drawbacks of the prior art to be eliminated.

Within the scope of this technical task, one object of the invention is to treat a milk or a plant-based beverage with predictable, repeatable results, irrespective of the type of milk or plant-based beverage treated.

Another object of the invention is to obtain, in the cup, a milk beverage or plant-based beverage with improved organoleptic properties, consistency and amount of froth, irrespective of the type of milk or plant-based beverage treated.

Another object of the invention is to provide a method for treating milk or a plant-based beverage that can be carried out automatically without any specific knowledge or abilities being required of the operator.

The technical task, as well as these and other objects, are achieved according to the present invention by providing a computer implemented method for heating and/or frothing milk according to claim 1. Said method comprises the following steps:
- providing in a memor of a controller a database containing associations between combinations of values of content by weight of proteins and carbohydrates of the milk or plant-based beverage to be treated and corresponding values of at least one treatment parameter for the milk or plant-based beverage;
- acquiring the content by weight of proteins and carbohydrates of the milk or plant-based beverage to be treated; and
- heating and /or frothing the milk or plant-based beverage with the value of said at least one treatment parameter corresponding in the database to the acquired values of the content by weight of proteins and carbohydrates.

In one embodiment of the invention, said acquisition step includes a spectrophotometric determination performed directly on said milk or plant-based beverage to be treated.

In a further embodiment of the invention, said acquisition step includes an automatic optical reading of information provided on a package of said milk or plant-based beverage to be treated. In a further embodiment of the invention, said acquisition step includes a personal acquisition of information.

The method in accordance with the invention makes it possible to obtain predictable and repeatable results when the type of milk or plant-based beverage used changes.

In particular, the milk for preparing a beverage can be of animal origin - whole, skimmed or partly skimmed - or plant origin, for example, from soy, rice, almond, oats, coconut, etc.

The milk or plant-based beverage to be treated is identified through a determination of the content by weight of proteins and carbohydrates and the treatment parameters for the milk or plant-based beverage are modulated according to the data determined so as to obtain in the cup substantially the same result when the type of milk or plant-based beverage used changes.

Optimal frothing and/or heating can be achieved almost automatically without relying on any specific experience of the consumer, who is required only to press a pushbutton on the control panel of a coffee machine or a milk frothing device.

In one embodiment of the invention, said database comprises a first association, wherein a first value of said at least one treatment parameter corresponds to a content by weight of proteins no lower than a first threshold value and a content by weight of carbohydrates no higher than a second threshold value.

In one embodiment of the invention, said database comprises a second association, wherein a second value of said at least one treatment parameter corresponds to a content by weight of proteins lower than said first threshold value and to a content by weight of carbohydrates higher than said second threshold value.

In one embodiment of the invention, said database comprises a third association, wherein a third value of said at least one treatment parameter corresponds to a content by weight of proteins higher than said first threshold value and to a content by weight of carbohydrates higher than said second threshold value, or to a content by weight of proteins lower than said first threshold value and to a content by weight of carbohydrates lower than said second threshold value.

In one embodiment of the invention, said at least one treatment parameter comprises at least the amount of thermal energy to be transferred overall and/or in the unit of time to the milk or plant-based beverage to be treated.

In one embodiment of the invention, said at least one treatment parameter further comprises at least the amount of air to be transferred overall and/or in the unit of time to the milk or plant-based beverage to be treated.

The present invention also discloses a device, particularly a coffee machine or a milk frothing device, comprising a receptacle for a milk or plant-based beverage to be treated, a station for said receptacle, a means for heating the milk or plant-based beverage, and a means for frothing the milk or plant-based beverage, characterised in that it has a spectrophotometer configured to determine the content by weight of proteins and carbohydrates of the milk or plant-based beverage to be treated present in said receptacle, and in that it has an electronic controller having in its memory a database containing associations between combinations of values of content by weight of proteins and carbohydrates of the milk or plant-based beverage to be treated and corresponding values of at least one treatment parameter for the milk or plant-based beverage, said controller being configured to control the heating and/or frothing of the milk or plant-based beverage with the value of said at least one treatment parameter corresponding, in the database, to the determined values of the content by weight of proteins and carbohydrates.

Preferably, the device also has a temperature sensor for sensing the temperature of the milk or plant-based beverage to be treated present in said receptacle.

Other features of the present invention are defined, moreover, in the subsequent claims.

Additional features and advantages of the invention will become more apparent from the description of a preferred but not exclusive embodiment of the method for treating milk or a plant-based beverage according to the invention, illustrated by way of non-limiting example in the appended drawings, in which:
figure 1 shows a first embodiment of a coffee machine suitable for implementing the method of the invention;
figure 2 shows a second embodiment of a coffee machine suitable for implementing the method of the invention;
figure 3 shows a graph of the values determined by the spectrophotometer when the types of milk used change.

With reference to the aforesaid figures, they show an automatic device, particularly a coffee machine, denoted in its entirety by the reference number 1, and specifically configured to treat a milk or a plant-based beverage.

The coffee machine 1 is of the type generically comprising a water circuit (not shown) having, in a cascade arrangement, a water reservoir or a connection to a water main, a water supply pump, a boiler for heating the water, a coffee brewing unit, and an external dispenser of the coffee extract.

The coffee machine 1 further comprises a station 2 for a receptacle 3 for containing milk or a plant-based beverage, a means 4 for heating the milk or plant-based beverage and a means 5 for frothing the milk or plant-based beverage.

The heating means 4 can be of the steam type or an electric heating element.

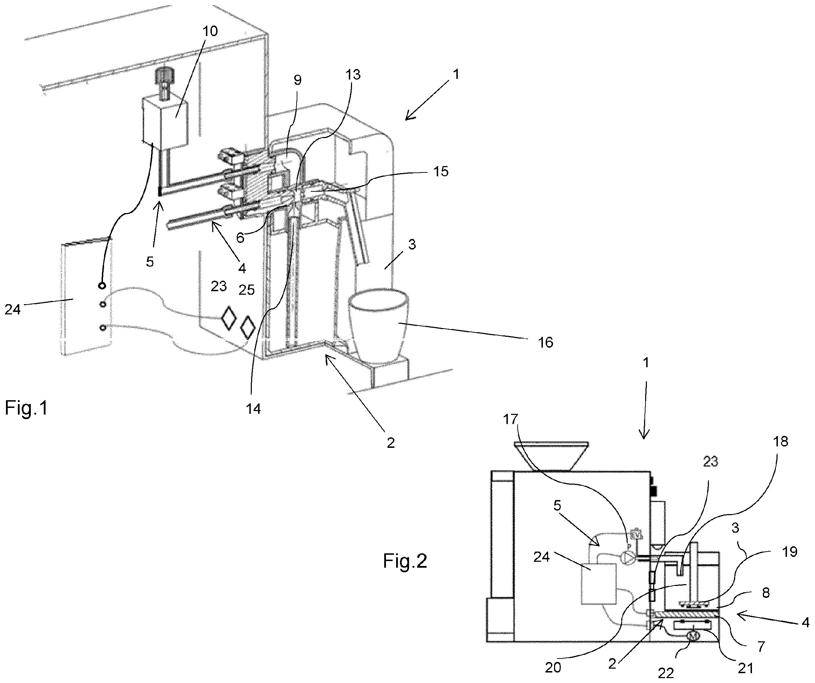

With reference to the solution shown in figure 1, the heating means 4 includes a steam dispenser 6 associated with a source of steam (not shown), for example a boiler supplied by a water pump. Again with reference to the solution shown in figure 1, the frothing means 5 includes an air conduit 9 fitted with a progressively opening valve 10. The air conduit 9 is connected to a Venturi effect mixing chamber 13. Also connected to the mixing chamber 13 there is a conduit 14 for the milk or plant-based beverage, the conduit extending inside the receptacle 3 and the steam dispenser 6, which, by dispensing a flow of steam, triggers the Venturi effect whereby air and the milk or plant-based beverage are drawn into the mixing chamber 13. The mixing chamber 13 is also connected to a conduit 15 for dispensing into a cup 16.

With reference to the solution shown in figure 2, by contrast, the heating means 4 includes an electric heater 7, for example incorporated in a base 8 of the station 2 for the receptacle 3.

Again with reference to the solution shown in figure 2, the frothing means 5 includes an air pump 17 having a dispensing conduit 18 which extends inside the receptacle 3, and a mechanical stirrer 19 present on the bottom of the receptacle 3. The mechanical stirrer 19 is supported in free rotation at the lower end of a vertical tube 20 supported by the receptacle 3. The vertical tube 20 has an upper end that projects from the receptacle 3 and bends downwards to dispense into a cup. The receptacle 3 is hermetically sealed by a removable lid so that the air pressure generated by the air pump 17 inside the receptacle 3 can push the milk or plant-based beverage upwards through the vertical tube 20 in order to be dispensed into a cup. The actuation of the mechanical stirrer 19 in rotation is of the magnetic type: more precisely, the mechanical stirrer 19 has magnetic or ferromagnetic parts configured and arranged so as to interact magnetically with permanent magnets distributed around the axis of a rotor 21 that can be driven in rotation by a motor 22. The rotor 21 is positioned below the station 2 of the receptacle 3 coaxially with the vertical tube 20.

The coffee machine can also have a heating means 4 and a frothing means 5 that are different from the ones illustrated thus far.

Therefore, irrespective of the specific construction of the heating means 4 and frothing means 5, the coffee machine 1 can advantageously have a spectrophotometer 23 configured to determine the content by weight of proteins and carbohydrates of the milk or plant-based beverage to be treated which is present in the receptacle 3.

The spectrophotometer 23 is based, in particular, on reflectance spectroscopy, which measures the spectral reflectance factor as a function of the wavelength of the incident radiation that is in the wavelength of the near infrared, visible and ultraviolet regions.

The coffee machine 1 has an electronic controller 24, to which the spectrophotometer 23, the heating means 4 and the frothing means 5 are connected.

The electronic controller 24 has in its memory a database containing associations Ak between combinations of values Sx, Sy of content by weight of proteins and carbohydrates of the milk or plant-based beverage to be treated and corresponding values Vi of at least one treatment parameter Pj for the milk or plant-based beverage to be treated.

Preferably, the coffee machine 1 also has a temperature sensor 25 for the determination, on contact or remotely, of the temperature of the milk or plant-based beverage to be treated which is present in the receptacle 3, for example an infrared temperature sensor outside the receptacle 3. The temperature sensor 25 is also naturally connected to the electronic controller 24.

The result of the spectrophotometer 23, which can depend on the temperature of the milk or plant-based beverage, can thus be normalised by the temperature data of the milk or plant-based beverage detected by the temperature sensor 25.

The temperature sensor 25 and the spectrophotometer 23 can be integrated into a single device configured for an acquisition from a same region inside the receptacle 3.

In this manner, the accuracy with which the reading of the spectrophotometer 23 is normalised can be improved.

The database comprises a first association A1, wherein a first value V1 of at least one treatment parameter Pj for the milk or plant-based beverage corresponds to a content by weight of proteins no lower than a first threshold value S1 and a content by weight of carbohydrates no higher than a second threshold value S2.

The database further comprises a second association A2, wherein a second value V2 of at least one treatment parameter Pj for the milk or plant-based beverage corresponds to a content by weight of proteins lower than the first threshold value S1 and to a content by weight of carbohydrates higher than the second threshold value S2.

Finally, the database comprises a third association A3, wherein a third value V3 of at least one treatment parameter Pj for the milk or plant-based beverage corresponds to a content by weight of proteins higher than the first threshold value S1 and to a content by weight of carbohydrates higher than the second threshold value S2, or to a content by weight of proteins lower than the first threshold value S1 and to a content by weight of carbohydrates lower than the second threshold value S2.

The treatment parameters for the milk or plant-based beverage that can be adjusted based on the reading of the spectrophotometer 23 include the amount of thermal energy which the heating means 4 transfers overall and/or in the unit of time to the milk or plant-based beverage to be treated and/or the amount of air which the frothing means 5 transfers overall and/or in the unit of time to the milk or plant-based beverage to be treated.

It has been found to be convenient to set the first threshold value S1 between 0.1% and 0.5% by weight, preferably equal to 0.5% by weight, and the second threshold value S2 between 7% and 15% by weight, preferably equal to 7% by weight.

The treatment method thus comprises acquiring, in particular, in the case illustrated, determining by means of the spectrophotometer, the content by weight of proteins and carbohydrates of the milk or plant-based beverage to be treated and treating the milk or plant-based beverage with the value V1, V2, V3, of the treatment parameter Pj or parameters Pj corresponding, in the database, to the combination of the determined value Sx of the content by weight of proteins and the determined value Sy of the content by weight of carbohydrates.

Examples are provided below of adjustments that may be made with the coffee machine illustrated in figure 1 to produce hot frothed milk in a cup when the type of milk used changes.

Tables 1 and 2 below refer to the adjustment of treatment parameters for three types of milk indicated as #1, #2 and #3, made with the heating means 4 and the frothing means 5.

Table 1 refers to the adjustment of the value Vi of the temperature P1 of the steam flow entering the mixing chamber 13: with this adjustment one adjusts the amount of thermal energy to be transferred overall and in the unit of time to the milk to be treated.

**Table 1**

| | Sx | Sy | S1 | S2 | V1(P1) | V2(P1) | V3(P1) | A1 | A2 | A3 |
|---|---|---|---|---|---|---|---|---|---|---|
| # 1 | 0.8% | 4% | 0.5% | 7% | 120°C | | | x | | |
| #2 | 0.3% | 10% | 0.5% | 7% | | 130°C | | | x | |
| #3 | 1.5% | 9% | 0.5% | 7% | | | 110°C | | | x |

Table 2 refers to the adjustment of the value Vi of opening P2 (expressed as a % of the passage that is open relative to the maximum passage opening) of the valve 10: with this adjustment one adjusts the amount of air to be transferred overall and in the unit of time to the milk to be treated.

**Table 2**

| | Sx | Sy | S1 | S2 | V1(P2) | V2(P2) | V3(P2) | A1 | A2 | A3 |
|---|---|---|---|---|---|---|---|---|---|---|
| # 1 | 0.8% | 4% | 0.5% | 7% | 100% | | | x | | |
| #2 | 0.3% | 10% | 0.5% | 7% | | 50% | | | x | |
| #3 | 1.5% | 9% | 0.5% | 7% | | | 15% | | | x |

The adjustment can obviously regard other or different milk treatment parameters, for example the degree of moisture of the steam dispensed, the pressure of the steam dispensed, the duty cycle of the water pump that supplies the boiler for producing steam, etc.

Examples are provided below of adjustments that may be made with the coffee machine illustrated in figure 2 to produce hot frothed milk in a cup when the type of milk used changes.

Tables 3 and 4 below refer to the adjustment of treatment parameters for three types of milk indicated as #1, #2 and #3, made with the frothing means 5 and the heating means 4.

Table 3 refers to the adjustment of the value Vi of the rotation speed P3 (expressed in revolutions per minute) of the mechanical stirrer 19: with this adjustment one adjusts the amount of air to be transferred overall and in the unit of time to the milk to be treated.

**Table 3**

| | Sx | Sy | S1 | S2 | V1(P3) | V2(P3) | V3(P3) | A1 | A2 | A3 |
|---|---|---|---|---|---|---|---|---|---|---|
| # 1 | 0.8% | 4% | 0.5% | 7% | 600 rpm | | | x | | |
| #2 | 0.3% | 10% | 0.5% | 7% | | 800 rpm | | | x | |
| #3 | 1% | 9% | 0.5% | 7% | | | 300 rpm | | | x |

The adjustment can obviously regard other or different milk treatment parameters, for example the duty cycle of the air pump 17.

Table 4 refers to the adjustment of the value Vi of the activation time P4 (expressed in seconds) of the electric heater 7: with this adjustment one adjusts the amount of thermal energy to be transferred overall to the milk to be treated.

**Table 4**

| | Sx | Sy | S1 | S2 | V1(P4) | V2(P4) | V3(P4) | A1 | A2 | A3 |
|---|---|---|---|---|---|---|---|---|---|---|
| # 1 | 0.8% | 4% | 0.5% | 7% | 110sec | | | x | | |
| #2 | 0.3% | 10% | 0.5% | 7% | | 130sec | | | x | |
| #3 | 1.5% | 9% | 0.5% | 7% | | | 120sec | | | x |

As may be seen from the tables shown above, a first value V1 of the treatment parameters P1, P2, P3 and P4 for the milk is associated with the type of milk # 1 that has a content by weight of proteins no lower than the first threshold value S1 and a content by weight of carbohydrates no higher than the second threshold value S2, a second value V2 of the treatment parameters P1, P2, P3 and P4 for the milk is associated with the type of milk # 2 that has a content by weight of proteins lower than the first threshold value S1 and a content by weight of carbohydrates higher than the second threshold value S2, and a third value V2 of the treatment parameters P1, P2, P3 and P4 for the milk is associated with the type of milk # 3, which differs from both of the previous situations.

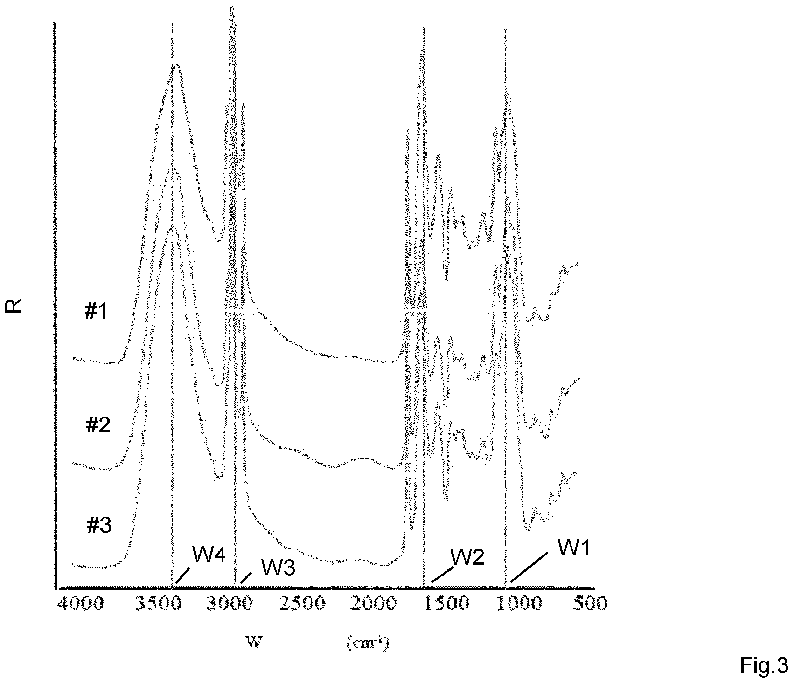

Figure 3 graphically represents the data typically acquired by the spectrophotometer 23.

The wavenumber W is shown on the x-axis and the reflectance R is shown on the y-axis for the three types of milk # 1, # 2 and # 3.

Peaks typically attributable to proteins, carbohydrates and fats can be noted: a first peak around the wavenumber W1 = 1000 cm⁻¹ related to the carbonyl group C=O of carbohydrates; a second peak around the wavenumber W2 = 1500 cm⁻¹ related to the carbonyl group C=O of carbohydrates and the amino group N-H of proteins; a third peak around the wavenumber W3 = 3000 cm⁻¹ related to the carbonyl group CH₂ of fats; and a fourth peak around the wavenumber W4 = 3500 cm⁻¹ related to the amino group N-H of proteins.

Thanks to the method disclosed by the present invention, the result in the cup can be optimised irrespective of the type of milk or plant-based beverage used.

The method advantageously provides for an automatic selection, from the database, of the correct treatment parameters for the milk or plant-based beverage.

The thermal energy and air input to the milk or plant-based beverage to be treated is modulated based on the type of milk or plant-based beverage so as to always obtain the best result in terms of organoleptic properties, volume, consistency and visual appearance of the froth of the product dispensed into the cup.

The modulation of the thermal energy input is particularly relevant, since the characteristics of different types of milk or plant-based beverages can degrade at different temperatures.

The spectrophotometer 23 can also be used to determine the nature of the liquid present in the receptacle 3.

For example, if the spectrophotometer 23 identifies the presence of water in the receptacle 3, the electronic controller 24 can automatically activate the performance of a cleaning treatment, which may be displayed, for example, on an interface of the coffee machine 1.

It should be noted that the treatment parameters which can be adjusted and the way in which they can be adjusted can be multiple and different from the ones illustrated.

Preferably, continual experimentation also on new commercially available types of milk and plant-based beverages can be provided for in order to update the database and reprogram the memory of the electronic controller with the updated database.

Finally, as mentioned, the method is applicable to any device, not necessarily a coffee machine, which has an electronic controller with a programmable memory, a heating means and a frothing means adjustable by the electronic controller, and optionally a spectrophotometer suitable for determining the content by weight of proteins and carbohydrates of the milk or plant-based beverage to be treated and communicating the data thus determined to the electronic controller for the purpose of adjusting the heating means and/or the frothing means.

The method for treating frothed milk thus conceived is susceptible of numerous modifications and variants as long as they are encompassed by the scope defined by the appended claims.

The scope of protection obviously extends to the treatment not only of milk, but also, as is evident from what was described above, of plant-based beverages.

The step of acquiring the content by weight of proteins and carbohydrates of the milk or plant-based beverage to be treated need not necessarily be carried out by means of a spectrophotometer.

In fact, the acquisition step can include an automatic optical reading of information shown on a package of the milk or plant-based beverage to be treated.

In such a case, the optical reading can be carried out by a mobile electronic device equipped with a camera and software for automatic recognition of information relating to the content by weight of proteins and carbohydrates of the milk or plant-based beverage to be treated, and the mobile electronic device can be connected by means of a wireless connection, for example a WiFi or Bluetooth connection, to a coffee machine or a milk frothing device that performs the treatment. In another case, the acquisition step can include a personal information acquisition step.

In such a case, the acquisition can comprise a step in which information relating to the content by weight of proteins and carbohydrates of the milk or plant-based beverage to be treated is manually uploaded by the user into the memory of the controller of the treatment device, either via a physical interface of the treatment device or by means of a mobile electronic device having a wireless connection, for example a WiFi or Bluetooth connection, to the treatment device.

The choice of the threshold values S1 and S2 can also be different from the one illustrated above, and in particular in some applications a first threshold value S1 higher than 1.5 % by weight and a second threshold value S2 higher than 7% by weight can be set.

The materials used, as well as the dimensions, may in practice be any whatsoever according to needs and the state of the art.

## Claims

1. A computer-implemented method for heating and/or frothing milk or a plant-based beverage, **characterised in that** it comprises the following steps:
- creating a database containing associations (Ak) between combinations of values (Sx, Sy) of content by weight of proteins and carbohydrates of the milk or plant-based beverage to be treated and corresponding values (Vi) of at least one treatment parameter (Pj) for the milk or plant-based beverage;
- acquiring the content by weight of proteins and carbohydrates of the milk or plant-based beverage to be treated; and
- heating and/or frothing the milk or plant-based beverage with the value (Vi) of said at least one treatment parameter (Pj) corresponding in the database to the acquired values (Sx, Sy) of the content by weight of proteins and carbohydrates.

2. The method for treating milk or a plant-based beverage according to claim 1, **characterised in that** said acquisition step includes a spectrophotometric determination performed directly on said milk or plant-based beverage to be treated.

3. The method for treating milk or a plant-based beverage according to claim 1, **characterised in that** said acquisition step includes an automatic optical reading of information provided on a package of said milk or plant-based beverage to be treated.

4. The method for treating milk or a plant-based beverage according to claim 1, **characterised in that** said acquisition step includes a personal acquisition of information.

5. The method for treating milk according to claim 1, **characterised in that** said database comprises a first association (A1), wherein a first value (V1) of said at least one treatment parameter (Pj) corresponds to a content by weight of proteins no lower than a first threshold value (S1) and a content by weight of carbohydrates no higher than a second threshold value (S2).

6. The method for treating milk according to the preceding claim, **characterised in that** said database comprises a second association (A2), wherein a second value (V2) of said at least one treatment parameter (Pj) corresponds to a content by weight of proteins lower than said first threshold value (S1) and to a content by weight of carbohydrates higher than said second threshold value (S2).

7. The method for treating milk according to the preceding claim, **characterised in that** said database comprises a third association (A3), wherein a third value (V3) of said at least one treatment parameter (Pj) corresponds to a content by weight of proteins higher than said first threshold value (S1) and to a content by weight of carbohydrates higher than said second threshold value (S2), or to a content by weight of proteins lower than said first threshold value (S1) and to a content by weight of carbohydrates lower than said second threshold value (S2).

8. The method for treating milk according to any one of claims 5 to 7, **characterised in that** said first threshold value (S1) is comprised between 0.1% and 0.5% by weight and said second threshold value (S2) is comprised between 7% and 15% by weight.

9. The method for treating milk according to the preceding claim, **characterised in that** said first threshold value (S1) is equal to 0.5% by weight and said second threshold value (S2) is equal to 7% by weight.

10. The method for treating milk according to any preceding claim, **characterised in that** said at least one treatment parameter (Pj) comprises at least the amount of thermal energy to be transferred overall and/or in the unit of time to the milk or plant-based beverage to be treated.

11. The method for treating milk according to any preceding claim, **characterised in that** said at least one treatment parameter (Pj) comprises at least the amount of air to be transferred overall and/or in the unit of time to the milk or plant-based beverage to be treated.

12. A device, particularly a coffee machine or a milk frothing device, comprising a receptacle (3) for a milk or plant-based beverage to be treated, a station (2) for said receptacle (3), a means (4) for heating the milk, and a means (5) for frothing the milk or plant-based beverage, **characterised in that** it has a spectrophotometer (23) configured and adapted to determine the content by weight of proteins and carbohydrates of the milk or plant-based beverage to be treated present in said receptacle (3), and **in that** it has an electronic controller (24) having in its memory a database containing associations (Ak) between combinations of values (Sx, Sy) of content by weight of proteins and carbohydrates of the milk or plant-based beverage to be treated and corresponding values (Vi) of at least one treatment parameter (Pj) for the milk or plant-based beverage to be treated, said controller (24) being configured to control the heating and/or frothing of the milk or plant-based beverage with the value (Vi) of said at least one corresponding treatment parameter (Pj) in the database at the determined values (Sx, Sy) of the content by weight of proteins and carbohydrates.

13. The device according to the preceding claim, **characterised in that** said at least one treatment parameter (Pj) includes at least the amount of thermal energy to be transferred overall and/or in the unit of time to the milk or plant-based beverage to be treated.

14. The device according to either of claims 12 and 13, **characterised in that** said at least one treatment parameter (Pj) further includes at least the amount of air to be transferred overall and/or in the unit of time to the milk or plant-based beverage to be treated.

15. The device according to the preceding claim, **characterised in that** it has a temperature sensor (25) for the determination, on contact or remotely, of the temperature of the milk or plant-based beverage to be treated present in said receptacle (3).

## Patentansprüche

1. Computerimplementiertes Verfahren zum Erhitzen und/oder Aufschäumen von Milch oder eines pflanzlichen Getränks, **dadurch gekennzeichnet, dass** es die folgenden Schritte umfasst:
- Erstellen einer Datenbank, die Zuordnungen (Ak) zwischen Kombinationen von Werten (Sx, Sy) des Gewichtsanteils von Proteinen und Kohlenhydraten der zu behandelnden Milch oder des pflanzlichen Getränks und entsprechenden Werten (Vi) von mindestens einem Behandlungsparameter (Pj) für die Milch oder das pflanzliche Getränk enthält;
- Erfassen des Gewichtsanteils von Proteinen und Kohlenhydraten der zu behandelnden Milch oder des pflanzlichen Getränks; und
- Erhitzen und/oder Aufschäumen der Milch oder des pflanzlichen Getränks mit dem Wert (Vi) des mindestens einen Behandlungsparameters (Pj), der in der Datenbank den erfassten Werten (Sx, Sy) des Gewichtsanteils von Proteinen und Kohlenhydraten entspricht.

2. Verfahren zur Behandlung von Milch oder eines pflanzlichen Getränks nach Anspruch 1, **dadurch gekennzeichnet, dass** der Erfassungsschritt eine spektrophotometrische Bestimmung umfasst, die direkt an der zu behandelnden Milch oder dem zu behandelnden pflanzlichen Getränk durchgeführt wird.

3. Verfahren zur Behandlung von Milch oder eines pflanzlichen Getränks nach Anspruch 1, **dadurch gekennzeichnet, dass** der Erfassungsschritt ein automatisches optisches Auslesen von auf einer Verpackung der zu behandelnden Milch oder des zu behandelnden pflanzlichen Getränks angegebenen Informationen umfasst.

4. Verfahren zur Behandlung von Milch oder eines pflanzlichen Getränks nach Anspruch 1, **dadurch gekennzeichnet, dass** der Erfassungsschritt eine persönliche Erfassung von Informationen umfasst.

5. Verfahren zur Behandlung von Milch nach Anspruch 1, **dadurch gekennzeichnet, dass** die Datenbank eine erste Zuordnung (Al) umfasst, wobei ein erster Wert (Vi) des mindestens einen Behandlungsparameters (Pj) einem Proteingewichtsanteil entspricht, der nicht unter einem ersten Schwellenwert (S1) liegt, und einem Kohlenhydratgewichtsanteil, der nicht über einem zweiten Schwellenwert (S2) liegt.

6. Verfahren zur Behandlung von Milch nach dem vorhergehenden Anspruch, **dadurch gekennzeichnet, dass** die Datenbank eine zweite Zuordnung (A2) umfasst, wobei ein zweiter Wert (V2) des mindestens einen Behandlungsparameters (Pj) einem Proteingewichtsanteil entspricht, der unter dem ersten Schwellenwert (S1) liegt, und einem Kohlenhydratgewichtsanteil, der über dem zweiten Schwellenwert (S2) liegt.

7. Verfahren zur Behandlung von Milch nach dem vorhergehenden Anspruch, **dadurch gekennzeichnet, dass** die Datenbank eine dritte Zuordnung (A3) umfasst, wobei ein dritter Wert (V3) des mindestens einen Behandlungsparameters (Pj) einem Proteingewichtsanteil entspricht, der über dem ersten Schwellenwert (S1) liegt, und einem Kohlenhydratgewichtsanteil, der über dem zweiten Schwellenwert (S2) liegt, oder einem Proteingewichtsanteil, der unter dem ersten Schwellenwert (S1) liegt, und einem Kohlenhydratgewichtsanteil, der unter dem zweiten Schwellenwert (S2) liegt.

8. Verfahren zur Behandlung von Milch nach einem der Ansprüche 5 bis 7, **dadurch gekennzeichnet, dass** der erste Schwellenwert (S1) zwischen 0,1 % und 0,5 % Gewichtsanteil liegt und der zweite Schwellenwert (S2) zwischen 7 % und 15 % Gewichtsanteil liegt.

9. Verfahren zur Behandlung von Milch nach dem vorhergehenden Anspruch, **dadurch gekennzeichnet, dass** der erste Schwellenwert (S1) gleich 0,5 % Gewichtsanteil und der zweite Schwellenwert (S2) gleich 7 % Gewichtsanteil ist.

10. Verfahren zur Behandlung von Milch nach einem der vorhergehenden Ansprüche, **dadurch gekennzeichnet, dass** der mindestens eine Behandlungsparameter (Pj) zumindest die Gesamtmenge der insgesamt und/oder pro Zeiteinheit auf die zu behandelnde Milch oder das zu behandelnde pflanzliche Getränk zu übertragenden thermischen Energie umfasst.

11. Verfahren zur Behandlung von Milch nach einem der vorhergehenden Ansprüche, **dadurch gekennzeichnet, dass** der mindestens eine Behandlungsparameter (Pj) zumindest die Gesamtmenge der insgesamt und/oder pro Zeiteinheit auf die zu behandelnde Milch oder das zu behandelnde pflanzliche Getränk zu übertragenden Luft umfasst.

12. Vorrichtung, insbesondere eine Kaffeemaschine oder ein Milchaufschäumer, umfassend ein Gefäß (3) für eine zu behandelnde Milch oder ein pflanzliches Getränk, eine Station (2) für das Gefäß (3), ein Mittel (4) zum Erhitzen der Milch und ein Mittel (5) zum Aufschäumen der Milch oder des pflanzlichen Getränks, **dadurch gekennzeichnet, dass** sie ein Spektrophotometer (23) aufweist, das so konfiguriert und angepasst ist, den Proteingewichtsanteil und den Kohlenhydratgewichtsanteil der in dem Gefäß (3) enthaltenen zu behandelnden Milch oder des pflanzlichen Getränks zu bestimmen, und dass sie einen elektronischen Regler (24) aufweist, in dessen Speicher eine Datenbank mit Zuordnungen (Ak) zwischen Kombinationen von Werten (Sx, Sy) des Proteingewichtsanteils und des Kohlenhydratgewichtsanteils der zu behandelnden Milch oder des pflanzlichen Getränks und entsprechenden Werten (Vi) von mindestens einem Behandlungsparameter (Pj) für die zu behandelnde Milch oder das pflanzliche Getränk enthalten ist, wobei der Regler (24) so konfiguriert ist, dass er das Erhitzen und/oder Aufschäumen der Milch oder des pflanzlichen Getränks mit dem Wert (Vi) des mindestens einen entsprechenden Behandlungsparameters (Pj) in der Datenbank bei den bestimmten Werten (Sx, Sy) des Proteingewichtsanteils und des Kohlenhydratgewichtsanteils steuert.

13. Vorrichtung nach dem vorhergehenden Anspruch, **dadurch gekennzeichnet, dass** der mindestens eine Behandlungsparameter (Pj) zumindest die Gesamtmenge der insgesamt und/oder pro Zeiteinheit auf die zu behandelnde Milch oder das zu behandelnde pflanzliche Getränk zu übertragenden thermischen Energie umfasst.

14. Vorrichtung nach einem der Ansprüche 12 und 13, **dadurch gekennzeichnet, dass** der mindestens eine Behandlungsparameter (Pj) ferner zumindest die Gesamtmenge der insgesamt und/oder pro Zeiteinheit auf die zu behandelnde Milch oder das zu behandelnde pflanzliche Getränk zu übertragenden Luft umfasst.

15. Vorrichtung nach dem vorhergehenden Anspruch, **dadurch gekennzeichnet, dass** sie einen Temperatursensor (25) aufweist, der zur Bestimmung der Temperatur der in dem Gefäß (3) enthaltenen zu behandelnden Milch oder des zu behandelnden pflanzlichen Getränks im Kontakt oder berührungslos ausgelegt ist.

## Revendications

1. Procédé mis en œuvre par ordinateur pour chauffer et/ou émulsionner du lait ou une boisson végétale, **caractérisé en ce qu'**il comprend les étapes suivantes :
- création d'une base de données contenant des associations (Ak) entre des combinaisons de valeurs (Sx, Sy) de la teneur en poids de protéines et de glucides du lait ou de la boisson végétale à traiter et des valeurs correspondantes (Vi) d'au moins un paramètre de traitement (Pj) pour le lait ou la boisson végétale ;
- acquisition de la teneur en poids de protéines et de glucides du lait ou de la boisson végétale à traiter ; et
- chauffage et/ou émulsion du lait ou de la boisson végétale avec la valeur (Vi) dudit au moins un paramètre de traitement (Pj) correspondant, dans la base de données, aux valeurs acquises (Sx, Sy) de la teneur en poids de protéines et de glucides.

2. Procédé de traitement du lait ou d'une boisson végétale selon la revendication 1, **caractérisé en ce que** ladite étape d'acquisition comprend une détermination spectrophotométrique réalisée directement sur ledit lait ou ladite boisson végétale à traiter.

3. Procédé de traitement du lait ou d'une boisson végétale selon 1a revendication 1, **caractérisé en ce que** ladite étape d'acquisition comprend une lecture optique automatique d'informations fournies sur un emballage dudit lait ou de ladite boisson végétale à traiter.

4. Procédé de traitement du lait ou d'une boisson végétale selon la revendication 1, **caractérisé en ce que** ladite étape d'acquisition comprend une acquisition personnelle d'informations.

5. Procédé de traitement du lait selon la revendication 1, **caractérisé en ce que** ladite base de données comprend une première association (A1), dans laquelle une première valeur (V1) dudit au moins un paramètre de traitement (Pj) correspond à une teneur en poids de protéines non inférieure à une première valeur seuil (S1) et à une teneur en poids de glucides non supérieure à une seconde valeur seuil (S2).

6. Procédé de traitement du lait selon la revendication précédente, **caractérisé en ce que** ladite base de données comprend une seconde association (A2), dans laquelle une seconde valeur (V2) dudit au moins un paramètre de traitement (Pj) correspond à une teneur en poids de protéines inférieure audit premier seuil (S1) et à une teneur en poids de glucides supérieure audit second seuil (S2).

7. Procédé de traitement du lait selon la revendication précédente, **caractérisé en ce que** ladite base de données comprend une troisième association (A3), dans laquelle une troisième valeur (V3) dudit au moins un paramètre de traitement (Pj) correspond à une teneur en poids de protéines supérieure audit premier seuil (S1) et à une teneur en poids de glucides supérieure audit second seuil (S2), ou à une teneur en poids de protéines inférieure audit premier seuil (S1) et à une teneur en poids de glucides inférieure audit second seuil (S2).

8. Procédé de traitement du lait selon l'une quelconque des revendications 5 à 7, **caractérisé en ce que** ledit premier seuil (S1) est compris entre 0,1 % et 0,5 % en poids et ledit second seuil (S2) est compris entre 7 % et 15 % en poids.

9. Procédé de traitement du lait selon la revendication précédente, **caractérisé en ce que** ledit premier seuil (S1) est égal à 0,5 % en poids et ledit second seuil (S2) est égal à 7 % en poids.

10. Procédé de traitement du lait selon l'une quelconque des revendications précédentes, **caractérisé en ce que** ledit au moins un paramètre de traitement (Pj) comprend au moins la quantité d'énergie thermique à transférer globalement et/ou par unité de temps au lait ou à la boisson végétale à traiter.

11. Procédé de traitement du lait selon l'une quelconque des revendications précédentes, **caractérisé en ce que** ledit au moins un paramètre de traitement (Pj) comprend au moins la quantité d'air à transférer globalement et/ou par unité de temps au lait ou à la boisson végétale à traiter.

12. Dispositif, en particulier une machine à café ou un dispositif pour émulsionner le lait, comprenant un récipient (3) destiné à contenir un lait ou une boisson végétale à traiter, une station (2) pour ledit récipient (3), un moyen (4) pour chauffer le lait, et un moyen (5) pour émulsionner le lait ou la boisson végétale, **caractérisé en ce qu'**il comporte un spectrophotomètre (23) configuré et adapté pour déterminer la teneur en poids de protéines et de glucides du lait ou de la boisson végétale à traiter présent dans ledit récipient (3), et **en ce qu'**il comporte un contrôleur électronique (24) ayant dans sa mémoire une base de données contenant des associations (Ak) entre des combinaisons de valeurs (Sx, Sy) de la teneur en poids de protéines et de glucides du lait ou de la boisson végétale à traiter et des valeurs correspondantes (Vi) d'au moins un paramètre de traitement (Pj) pour le lait ou la boisson végétale à traiter, ledit contrôleur (24) étant configuré pour commander le chauffage et/ou l'émulsion du lait ou de la boisson végétale avec la valeur (Vi) dudit au moins un paramètre de traitement (Pj) correspondant dans la base de données aux valeurs déterminées (Sx, Sy) de la teneur en poids de protéines et de glucides.

13. Dispositif selon la revendication précédente, **caractérisé en ce que** ledit au moins un paramètre de traitement (Pj) comprend au moins la quantité d'énergie thermique à transférer globalement et/ou par unité de temps au lait ou à la boisson végétale à traiter.

14. Dispositif selon l'une quelconque des revendications 12 ou 13, **caractérisé en ce que** ledit au moins un paramètre de traitement (Pj) comprend en outre au moins la quantité d'air à transférer globalement et/ou par unité de temps au lait ou à la boisson végétale à traiter.

15. Dispositif selon la revendication précédente, **caractérisé en ce qu'**il comporte un capteur de température (25) destiné à la détermination, par contact ou à distance, de la température du lait ou de la boisson végétale à traiter présent dans ledit récipient (3).
